(19) Europäisches Patentamt European Patent Office Office européen des brevets

(11) **EP 2 365 797 B1**

(12) **EUROPEAN PATENT SPECIFICATION**

(45) Date of publication and mention of the grant of the patent:
**02.04.2014 Bulletin 2014/14**

(51) Int Cl.:
*C12N 15/88* (2006.01)     *A61K 9/127* (2006.01)
*C12N 15/11* (2006.01)

(21) Application number: **09760130.6**

(22) Date of filing: **09.11.2009**

(86) International application number:
**PCT/EP2009/064848**

(87) International publication number:
**WO 2010/052326 (14.05.2010 Gazette 2010/19)**

(54) **SMALL INTERFERING RNA DELIVERY**

ABGABE VON SIRNA (SMALL INTERFERING RNA)

ADMINISTRATION DE PETIT ARN INTERFÉRENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **08.11.2008 EP 08168696**

(43) Date of publication of application:
**21.09.2011 Bulletin 2011/38**

(73) Proprietor: **Lipoxen Technologies Limited London NW1 0NH (GB)**

(72) Inventors:
  • **BACON, Andrew David**
    **London NW1 0NH (GB)**
  • **LAING, Peter**
    **London NW1 0NH (GB)**
  • **GREGORIADIS, Gregory**
    **London NW1 0NH (GB)**

(74) Representative: **Schlich, George et al**
    **Schlich LLP**
    **9 St Catherine's Road**
    **Littlehampton**
    **West Sussex BN17 5HS (GB)**

(56) References cited:
WO-A-2006/113679     US-A1- 2007 172 430
US-A1- 2008 063 701     US-A1- 2008 089 932

• PONS M ET AL: "LIPOSOMES OBTAINED BY THE ETHANOL INJECTION METHOD" INTERNATIONAL JOURNAL OF PHARMACEUTICS, ELSEVIER BV, NL, vol. 95, no. 1/03, 1 January 1993 (1993-01-01), pages 51-56, XP000960991 ISSN: 0378-5173
• BATZRI S ET AL: "SINGLE BILAYER LIPOSOMES PREPARED WITHOUT SONICATION" BIOCHIMICA ET BIOPHYSICA ACTA, AMSTERDAM, vol. 298, 1 January 1973 (1973-01-01), pages 1015-1019, XP000878589 ISSN: 0006-3002
• MAITANI Y ET AL: "Effect of sugars on storage stability of lyophilized liposome/DNA complexes with high transfection efficiency" INTERNATIONAL JOURNAL OF PHARMACEUTICS, ELSEVIER BV, NL, vol. 356, no. 1-2, 22 May 2008 (2008-05-22), pages 69-75, XP022625172 ISSN: 0378-5173 [retrieved on 2007-12-31]

SEQUENCE LISTING

<110> Lipoxen Technologies Limited Bacon, Andrew D Laing, Peter Gregoriadis, Gregory

<120> Small Interfering RNA Delivery

<130> P39619WO

<140> PCT/EP2009/064848
<141> 2009-11-09

<150> EP 08168696.6
<151> 2008-11-08

<160> 4

<170> PatentIn version 3.5

<210> 1
<211> 21
<212> RNA
<213> Artificial

<220>
<223> synthetic ribonucleic acid sequence

<400> 1
gucaucacac ugaauaccaa u          21

<210> 2

<211> 23
<212> RNA
<213> Artificial

<220>
<223> synthetic ribonucleic acid sequence

<400> 2
auugguauuc agugugauga cac        23

<210> 3
<211> 21
<212> RNA
<213> Artificial

<220>
<223> synthetic ribonucleic acid sequence

<400> 3
gugaucagac ucaauacgaa u        21

<210> 4
<211> 23
<212> RNA
<213> Artificial

<220>
<223> synthetic ribonucleic acid sequence

<400> 4
auucguauug agucugauca cac        23

**Description**

The present invention relates to a new delivery vehicle for small interfering RNA (siRNA), specifically a liposomal delivery system.

Small interfering RNA (or short interfering RNA) is a double stranded duplex that silences target gene expression through the process of RNA interference. The duplex surrogates into the natural process of gene silencing in a process in which the duplex unwinds, one of the strands becomes associated with a target mRNA molecule in a complex named the RNA induced silencing complex (RISC) that leads to destruction of the mRNA. siRNA has the potential to down regulate gene expression *in vivo.*

One model gene knockdown assay system involves introduction of an exogenous β-galactosidase reporter gene introduced endogenously into cell cultures. Spagnou et al., (2004) Biochemistry, 43, 13348-13356 describes in *vitro* transfection of siRNA targeting β-galactosidase, using cationic liposomal complexation. The authors describe the complexes in comparable terms to well known DNA/lipid complexes, as Lipoplex particles. However Spagnou *et al.*, explain that siRNA, being small subnanometric particles, may not be adequately protected from enzymatic or physical degradation by such complexation. They nevertheless use formulation techniques using physical admixture of empty SUVs formed of cationic/neutral lipid mixtures with siRNA.

Judge, et al., in Molecular Therapy 2006, 13(3) 494-505, investigate methods of entrapment of siRNA to protect the subject of siRNA therapy from side effects from unwanted innate immune responses to the siRNA material which is liable to activate toll-like receptors and stimulate type-I interferon production, as well as to enhance cellular uptake of the siRNA. Judge, *et al*., show that immunostimulatory activity of siRNA may be reduced by modifications to one strand of the duplex, for instance the chemical modification of the ribosyl 2'-OH group by methylation or fluorination. Judge, *et al.*, uses entrapment in liposomes in order to protect siRNA against nuclease digestion *in vivo.* They use siRNA specific for apolipoproteinB (apoB), which had previously been shown to knock down selectively the expression of apoB in the liver. The encapsulated siRNA was delivered intravenously to mice and showed that serum apoB levels, and thereby apoB bound cholesterol were reduced, despite symptoms of toxicity with unmodified siRNA duplexes illustrated by weight loss 1-3 days after administration. The encapsulating liposomes were formed of a mixture of cationic and neutral and PEGylated lipids. Judge, *et al.*, achieved reduced toxicity by methylation of ribosyl groups in some of the ribose residues of one strand of the siRNA duplex.

We have previously described the entrapment of gene vaccines into the intravesicular space of liposomes, and shown that this achieves useful protective results. We have used liposomes formed of cationic and neutral lipids in combination, as well as neutral liposomes. In WO9810748 we show that better immune response is achieved using cationic liposomes than neutral liposomes. We use the dehydration/rehydration vesicle (DRV) technique of liposomal entrapment, originally described by Kirby and Gregoriadis, et al., in Bio/technology 1984, 979-984.

We have also described liposomal co-entrapment of peptide antigen and gene vaccine, encoding a peptide antigen sharing at least one and preferably several epitopes with the peptide antigen in W02004004758. We used the DRV method, using cationic liposomes.

The DRV method involves a process in which empty small unilamellar liposomes (SUVs) having average diameter up to around 100 nm, are admixed with an aqueous solution of active to be entrapped, the mixture is dehydrated, and is subsequently rehydrated. The liposomes subsequently formed have active entrapped in the intravesicular space of liposomes having a higher average diameter than the starting SUVs. We have shown that the sizes of the liposomes may be controlled by inclusion of sugar in the SUV/active mixture, in WO9965465. We have shown that this technique is of utility for gene vaccine and peptide antigen co-entrapment for hepatitis B surface antigen and influenza HA.

Unlike vaccination, where exposure to lipid vehicles is a transient, perhaps once in a lifetime experience, the disease indications for siRNA include chronic diseases, wherein chronic dosing would be expected. It has been shown that repeated administration of cationic lipids could have adverse effects see below.

Landen, et al., in Cancer Research 2005, 65(15) 6910-6918, describe incorporation of siRNA targeting the oncoprotein EphA2 into liposomal vehicles and showed the product to be highly effective in reducing *in vivo* EphA2 expression in a mouse model of ovarian cancer. The liposomes were formed of dioleoyl phosphatidylcholine, which showed a 10 fold improvement of delivery over liposomes formed of a cationic lipid. Landen, *et al.*, point out that cationic lipid is preferentially taken up by the liver and spleen, which may limit its effectiveness in systemic therapy. They conclude that DOPC balances efficient uptake of siRNA into a liposome at preparation, uptake of the liposome into a cell and breakdown of the intracellular liposome with release of siRNA into the cytoplasm. Later work published in Halder, et al., Clin. Cancer Res. 2006, 12(16) 4916-4924 describe liposomal entrapment in similar liposomes of siRNA targeting focal adhesion kinase, again for ovarian cancer treatment. Again it is pointed out that cationic liposomes have toxicity problems in that they promote pulmonary inflammation, as well as having limited success in siRNA delivery, believed to be due to formation of excessively stable liposomes which do not disintegrate intracellularly to release the siRNA.

Landen, *et al.*, and Halder, *et al.*, use a liposomal entrapment technique involving tertiary butanol and tween based admixture of lipid and active, followed by lyophilisation then rehydration. They do not disclose the sizes of the liposomes. It is important for the alcohol to be removed but this can be difficult and is inconvenient. It is also desirable but technically difficult to remove the tween.

US 2007/0172430 (Brito et al) describes preparations of liposomes formulated as dry powders for mucosal, intranasal, inhalation or pulmonary delivery which may include one or more siRNAs or dicer-active precursors thereof targeted to a transcript involved in infection by, or replication or production of an influenza virus.

Pons et al. (International Journal of Pharmaceutics, 95 (1993) 51-56) describes liposomes obtained by using variants of the ethanol injection method and encapsulation of the drugs ciprofloxacin and enrofloxacin therein.

Batzri and Korn (Biochimica et Biophysica Acta, 298 (1973) 1015-1019) describes the production of liposomes by injecting an ethanolic solution of phospholipid into water. The resulting liposomes are indistinguishable from liposomes obtained by sonication.

WO 2006/113679 (Board of Regents, The University of Texas System) describes the encapsulation of siRNA molecules in liposomes for delivery to a cell. The liposomes were produced by mixing phospholipid with siRNA, butanol and Tween-20 by a method described by Landen et al., above.

Maitani et al. (International Journal of Pharmaceutics 356 (2008) 69-75) describes the production of lyophilized DNA-containing liposomes by a method which uses a lipid/sugar mixture. Maitani et al. describe the effect of varying the sugar : lipid ratio and the type of sugar used on the storage stability of the lyophilized DNA-containing liposomes.

US 2008/0063701 (Keller et al.) describes the production and use of cationic liposomes for transferring siRNA into cells.

US 2008/0089932 (Panzner et al.) describes the preparation of amphoteric liposomes composed of a mixture of lipids and methods of predicting the fusogenicity of an amphoteric liposome at a given pH. Also disclosed are methods of encapsulating siRNA in such amphoteric liposomes.

According to the invention we provide a new method for producing liposomal siRNA composition comprising double stranded siRNA entrapped in liposomes comprising the steps:

i) providing an aqueous suspension of empty liposomes having an average diameter in the range 20 to 200 nm and formed of neutral liposome forming components;

ii) mixing into the suspension double-stranded siRNA and sugar in an amount in the range 0.1 to 10.0% by weight based on the weight of liposome-forming components to form a mixed suspension, and in which the weight ratio of sugar to liposome forming components is in the range 0.75:1 to 7:1; and

iii) dehydrating the mixture formed in step ii) to form a dehydrated composition,

wherein liposomes formed by rehydrating the dehydrated composition have an average diameter in the range 100 to 500 nm and wherein the siRNA and sugar are each entrapped in the intravesicular space of the liposomes.

In the invention the siRNA may be modified so as to stabilise against RNase activity, or to reduce its toxicity, for instance as disclosed by Judge, et al., by modification of the 2'-hydroxyl groups of ribosyl moieties. However an advantage of the present invention is that the liposomal entrapment appears to reduce toxicity of the siRNA evidenced by minimising weight loss in treated animals, even when using unmodified siRNA. Preferably therefore in the invention the siRNA is not modified, thereby avoiding extra modification steps.

Liposomes administered into an animal parenterally ie by injection, are taken up by certain cells avidly. Such cells are mostly those of the reticular endothelial system (RES), for example liver cells (Kuppfer cells), and other macrophages in the spleen, bone marrow, lungs, blood (e.g. monocytes, neutrolfills), etc. Uptake by these cells is facilitated by plasma opsonins which coat liposomes, thus rendering them "edible" by the macrophages. This is usually referred to as passive targeting of liposomes. In the case of the liver, liposomes smaller than 100 nm average size, can reach the hepatic parenchymal cells through the 100 nm fenestrations. Liposomes given by the subcutaneous or intramuscular route, end up to a significant extent in the draining lymph nodes. With liposomes given orally, these are usually designed to remain stable in the gut. They are eventually taken up by M cells and the Peyer's patches. We believe that the liposomes produced by the method of the invention will behave in this generally observed manner.

We have established that the sugar-containing liposomes deliver the siRNA to the liver. The siRNA is suitably for treatment of a disease where the liver is the target organ, i.e. where the gene to be silenced is normally expressed. In the invention the siRNA may target chronic diseases such as cardiovascular disease, hepatitis due to viral infection (e.g. hepatitis B, C), cirrhosis of the liver caused by viral infection or alcoholism, chronic viral infections such as HIV/AIDS and HTLV-I/tropical spastic paraperesis, blood coagulation disorders such as thrombosis, metabolic disorders such as type-I and type-II diabetes, obesity, rheumatoid arthritis, osteoarthritis, systemic lupus erythematosus, neoplastic diseases (cancer etc.), osteoporosis etc.

If the target is cells having no specific affinity for liposomes, the liposomes made in the invention can be coated with ligands (e.g. antibodies, desialylated peptides or proteins) which bind to the corresponding receptors on the cell's surface and are usually interiorised together with the liposome moiety. This is usually referred to as active targeting.

The siRNA is suitably present in an amount in the range 0.1 to 10.0% by weight based on the weight of liposome forming components.

The product of the method may be a composition in dry form. Alternatively it may be in the form of an aqueous suspension in which the siRNA and sugar are each entrapped in the intravesicular space of the liposomes.

Other preferred embodiments of this aspect of the invention are mentioned in the claims.

On rehydration of the dried material from step iii), liposomes, encapsulating the siRNA are formed. The increase in size of the liposomes thus obtained as compared to the liposomes obtained in step i) is much lower when compared to the liposome in preparations which do not include a sugar. The need for further extrusion, microfluidisation or homogenisation steps may thus avoided. Such further steps may be damaging to the siRNA, indeed we have shown previously in that DNA nucleic acid material is substantially damaged by microfluidisation. Sometimes additional size reduction steps may be desired.

A preferred embodiment of the present invention includes the step of rehydration of the product of step iii).

Thus this method gives rise to the possibility of obtaining small highly loaded vesicles or liposomes, which may be particularly useful in the formation of pharmaceutical compositions.

It is particularly suitable however for the production of small liposomes for pharmaceutical use. For this purpose, the liposomes obtained in step i) are suitably small unilamellar vesicles with an average size, for example in the range of from 25 nm to 90 nm, preferably in the range from 50 to 90 nm and conveniently from 70 to 90 nm. Liposomes obtained ultimately from the process of the invention will still be small, with average size of less than 500 nm, usually from 100-200 nm.

The liposomes preferably have a low polydispersity index, that is they have relatively uniform sizes. The PD should preferably be at least 0.2 preferably at least 0.5. The inventors believe that the uniformity of size, as well as the relatively small size of the product, rehydrated liposomes allows them to have long half-life in the circulation and furthermore be delivered to the target organs. The size allows transcapillary passage. The can, for instance reach the lymph nodes or inflamed tissue, where they can be incorporated into cells and the siRNA delivered.

The liposomes used in step i) are empty liposomes, obtained by any of the conventional methods, for example using a classical method as described above. Any liposomes which are produced which have an average size which is too large for the desired purpose may be reduced for example using sonication, homogenisation, extrusion or microfluidisation techniques as are known in the art.

Lipids used in the production of the liposomes are well known in the art. They include, for example, phosphatidylcholines (PC), for instance dioleoyl PC, dipalmitoyl phosphatidylcholine (DPPC) or distearoyl phosphatidylcholine (DSPC), phosphatidyl ethanolamines, and/or cholesterol (CHOL). The selection of lipid will depend, to some extend on the nature of the active agent and the intended purpose of the liposome.

The sugar may be predissolved in water before being added to the empty liposomes or may be added as a solid to the suspension.

Suitable sugar for use in step ii) include aqueous solutions of monosaccharides such as glucose and fructose, disaccharides such as lactose or sucrose as well as polysaccharides. A particularly preferred sugar for use in the method of the invention is a disaccharide such as sucrose or lactose or a monosaccharide such as glucose. In particular, the sugar is sucrose.

Suitably the amount of sugar used in step ii) is such that the mass ratio of sugar to lipid is in the range of from 0.75:1 to 7:1, preferably 0.9:1 to 5:1, for instance the ratio may be 1:1 to 6:1 w/w, suitably from 1:1 to 5:1 w/w. it has been found that the greater the amount of sugar present, the lower the increase in size of the liposomes obtained following rehydration as compared to those obtained in step i). However, the degree of entrapment of the reagent may be lower. Thus the precise selection of ratios used will depend upon the required end use, with a balance being determined between the degree of entrapment for a given lipid content and liposome size. The difference this makes to the liposome formation varies to a certain extent, depending upon the particular reagent employed as discussed further below. Suitably, the amount of sugar present is less than 10% w/v of the composition.

It has further been found that increasing the volume of the sugar solution used in the process, by reducing the concentration of the sugar solution, may enhance entrapment. Suitable concentrations of sugar solutions are from 20 to 200 mM, preferably from 30 to 150 mM.

In addition, it has been found that if the subsequent rehydration is effected at elevated temperatures, for example of from 30 to 80°C, in particular from 40 to 65°C and especially at about 60°C, entrapment values can be increased. This has been found to be effective with liposomes comprising PC and CHOL, which would usually be formed at room temperature. There may be some size increase as compared to the starting liposomes when using elevated temperatures in this way, and therefore, this should be taken into account in selecting the particular conditions used to produce liposomes in any particular case.

The selection of conditions which will give liposomes of the desired size and loading, including the sugar: lipid mass ratio, the selection of lipid, the concentration of the sugar solution used, the amount of siRNA included in the solution, and the temperature of rehydration, can be determined using routine methods.

The drying step iii) above may be carried out using conventional methods, for example by freeze drying, spray drying, flash crystallisation, air stream drying (for example on a fluidised bed), vacuum drying, oven drying or any other method known in the art. Although the mechanical properties of the products of these two processes may be different, with the product of a spray drying process being a discrete and frequently flowable powder, and freeze drying producing a solid cake, the properties of the liposomes on rehydration in terms of their stability and entrapment is broadly similar.

For many applications, including the production of pharmaceutical compositions, spray drying may be preferable for step iii) as a result of the suitability of the mechanical properties of the product for further processing.

The product of freeze-drying comprises a block of porous cake which has relatively poor mechanical properties. The use of jet milling of the cake to achieve better properties can be effected, but damage can occur in this additional step.

Spray drying can achieve a dry product with good mechanical properties than can be delivered by inhalation, or reconstituted in water and administered by the parenteral route.

The subsequent rehydration step, step iv) may be carried out during the manufacture process or alternatively, the composition may be supplied in the dry state and rehydrated at the site of intended use, for example in the hospital or pharmacy where an encapsulated pharmaceutical is to be administered to patients.

Accordingly, an embodiment of the invention is a powder obtainable by the method of the invention.

The freeze-dried and rehydrated liposomes obtained have a good stability resulting in a long shelf life of the products.

The process of the invention allows high entrapment yields and levels (i.e. high amounts of siRNA based on liposome-forming components), which is highly advantageous where the product is to be used for repeated treatments.

Liposome products obtained using the above described method may be formulated as pharmaceutical compositions, for example by combining them with pharmaceutically acceptable carriers or excipients. The formulations may be suitable for parenteral in particular intravenous, or topical application, for example to the skin or to mucosal surfaces. A particular useful composition of the invention is a composition which is suitable for application intravenously, subcutaneously or intra-muscularly.

The compositions are generally formulated so as to be suitable for administration intravenously, although other techniques may also be used such as intramuscular or subcutaneous or orally or intravitreally. Depending on the disease to be treated, the compositions may be administered daily, several times per day, or less often, for instance weekly or by infusion over a period of n.

The present invention provides liposomes formed of ingredients which are naturally occurring in man and hence low overall toxicity. The liposomes successfully deliver their contents at the desired target, which is believed to be through good size control which allows delivery from the circulation to the target organs (where) without allowing the siRNA to give rise to cytokine responses.

The invention will now be particularly described by way of example with reference to the accompanying example, the results of which are illustrated in the accompanying drawings.

In the drawings:

Figure 1a and b show some results from Example 1;
Figure 2 shows further results from Example 1;
Figure 3 shows further results from Example 1; and
Figure 4 shows a reference gel indicating the electrophoresis of murine LDL using the kit for cholesterol quantification used in Example 1.


**Example 1 Demonstration of an effective siRNA liposomal formulation, in mice, following intravenous dosing siRNA within liposomes.**

Experimental details:-

Through out the experimental details described where RNA based component was present all other components were RNase free.

The siRNA duplex (drug) product/s were formed by annealing the following oligonucleotides (Gel purified (95-99% purity), desalted) (Table 1):-

**Table 1** Oligonucleotide and duplex details:-

| Oligo number | Sequence (5'-3') | Duplex product |
|---|---|---|
| 1 | GUCAUCACACUGAAUACCAAU | Duplex ID1 |
| 2 | *AUUGGUAUUCAGUGUGAUGACAC | |
| | | |

(continued)

| Oligo number | Sequence (5'-3') | Duplex product |
|---|---|---|
| 3 | GUGAUCAGACUCAAUACGAAU | Duplex ID2 |
| 4 | *AUUCGUAUUGAGUCUGAUCACAC | |
| * asterisks represent 5' phosphates | | |

Following annealing duplexes were stored <-40°C until use

SUV liposomes with an average size of 83.1 nm ± 11.9 (standard deviation) with a polydispersity index = 0.297 ± 0.071 (standard deviation), 0.22 μm sterile filtered composed of egg phosphatidyl choline (Lipoid, 99% PC) were prepared in H20 by emulsification of lipid mass followed by high pressure homogenisation (40,000 psi) at room temperature with a continuous nitrogen purge. The liposome formulation was prepared, on ice, by mixing the SUV liposomes with the siRNA duplex ID1 followed by the addition of dry sucrose. The mass ratio of the siRNA duplex : liposome SUV : sucrose components was 1 : 500 : 500. After sucrose solubilisation the formulation was freeze dried, as were the native duplexes ID1 and ID2.

Groups of 4 mice were injected intravenously, daily for a total of 3 days (thus a total of 3 doses) via the lateral tail vein with: - a) rehydrated from freeze dried, with water for injection, liposomal siRNA duplex ID1, b) rehydrated from freeze dried, with water for injection, siRNA duplex ID1, and c) rehydrated from freeze dried, with water for injection, siRNA duplex ID2. The duplex mass administered, identical for ID 1 and ID2, was 100 μg per dose thus 300 μg in total (3 doses). The total amount of PC used in the preparation for the 4 mice. Thus the amounts were 225 mg PC (as SUV liposomes prior to freeze drying), 225 mg sucrose and 450 micrograms duplex ID1. The final volume of the rehydrated preparation was 1.125 ml from which 0.25 ml was used to dose each mouse.

Liposome size after rehydration stage was determined by photon correlation spectroscopy (PCS, also known as Dynamic Light Scattering (DLS)) at 25°C using an Malvern Nano ZS (Malvern Instruments UK, model ZEN3600), equipped with a 4 mW 633 He-Ne laser. Z average mean diameter particle and size distribution (polydispersity index) values were obtained.

The entrapment efficiency of the siRNA duplex was determined by ultracentrifugation (~200,000 g, 1 hr) of the rehydrated liposomal formulation in wash buffer, repeated twice (each time with 7 m/wash buffer), followed by quantification of siRNA in wash buffer after concentrating by freeze drying and rehydrating in about 2% the original volume of water by UV spectophotometry (absorption at 260 and 280 nm, 1 cm path length, spectrophotometer model: - Hitachi U-2800A). A UV spectophotometry (absorption at 260 and 280 nm) standard curve of native duplex ID1 was used to quantify siRNA in wash buffer.

Serum samples were obtained from all mice; the day before intravenous dosing (day 0), after the second dose (day 3), two days after the third dose (day 5) and finally four days after the third (last) dose (day 7). The body weights of all mice were measured at 0, 3, 7, 10, and 14 day/s after the first dose was administered

The mouse serum samples thus obtained were stored chilled, on ice, until assayed for LDL / HDL cholesterol by electrophoresis in agarose gels using a Sebia HYDRASYS system (Sebia UK, River Court, The Meadows Business Park Blackwater, Camberley, GU17 9AB), utilising HYDRAGEL 7 HDL/LDL Chol Direct kit. Briefly, the HYDRAGEL 7 LDL/HDL CHOL Direct kits are intended for the quantification of the cholesterol carried by the different lipoprotein fractions separated on agarose gel electrophoresis on the HYDRASYS. Gels were stained specifically for the presence of cholesterol using cholesterol esterase in a chromogenic reaction. Integration of the optical density obtained from densitometric scanning of agarose gels after enzymatic staining, specific for cholesterol, allows direct and simultaneous quantification of the LDL and HDL fractions

Results:-

The particle size distribution by PCS of the rehydrated liposomal composition of siRNA duplex ID1 was, calculated from sample analysis in triplicate, an average 186.1 nm ± 17.94 (standard deviation) with a polydispersity index = 0.665 ± 0.123 (standard deviation).

% siRNA entrapment was calculated as 55.2 %, using the following calculation (Calculation 1):-

$$\% \text{ siRNA entrapment } = \frac{(\text{Total siRNA duplex} - \text{siRNA duplex in ultracentrifugation wash buffer})}{\text{Total siRNA duplex}}$$

$$\times 100$$

The absolute UV absorbance values of the siRNA duplex recovered in the ultracentrifugation wash buffer were: - 1.120 Abs at 260 nm (1cm path length) and 0. 558 at 280 nm (1 cm path length)

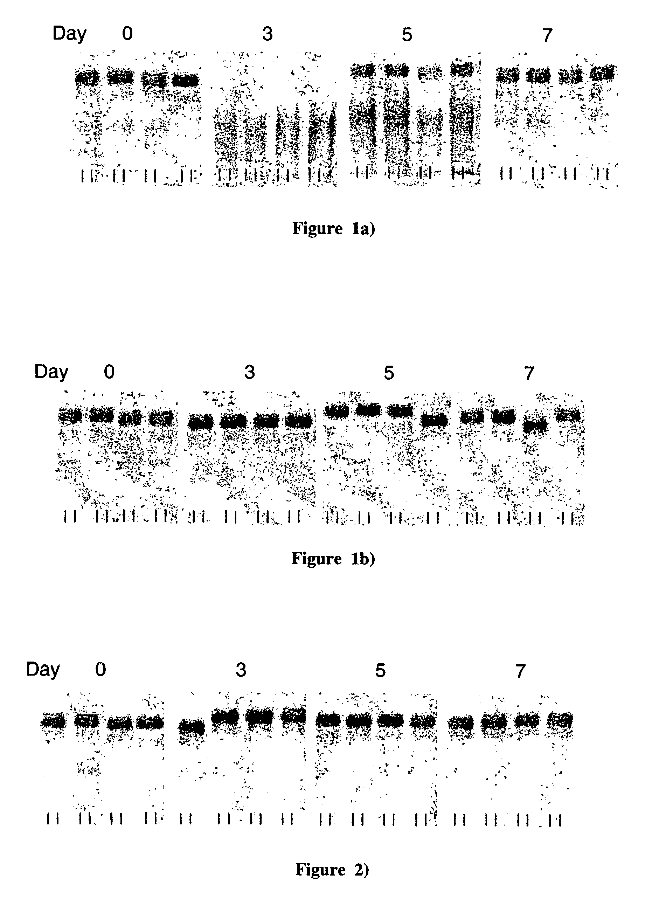

Figure 1 a) shows the densitometric scans of the agarose gels from the serum sample analysis from mice dosed with: - a) rehydrated from freeze dried, with water for injection, liposomal siRNA duplex ID1. Sera samples (four) from each sample bleed day were applied at the origin (marked by: - | | ) and separated by electrophoresis (migration is bottom of page to top). Each lane consists of the profile of a serum sample from an individual mouse.

Figure 1b) shows the densitometry scans of the agarose gels from the sera sample analysis from mice dosed with :- b) rehydrated from freeze dried, with water for injection, siRNA duplex ID1. Sera samples (four) from each sample bleed day were applied at the origin (marked by:- | | ) and separated by electrophoresis (migration is bottom of page to top). Each lane consists of the profile of a serum sample from an individual mouse

Figure 2) shows the densitometry scans of the agarose gels from the sera sample analysis from mice dosed with :- c) rehydrated from freeze dried, with water for injection, siRNA duplex ID2. Sera samples (four) from each sample bleed day were applied at the origin (marked by: - | | ) and separated by electrophoresis (migration is bottom of page to top). Each lane consists of the profile of a serum sample from an individual mouse.

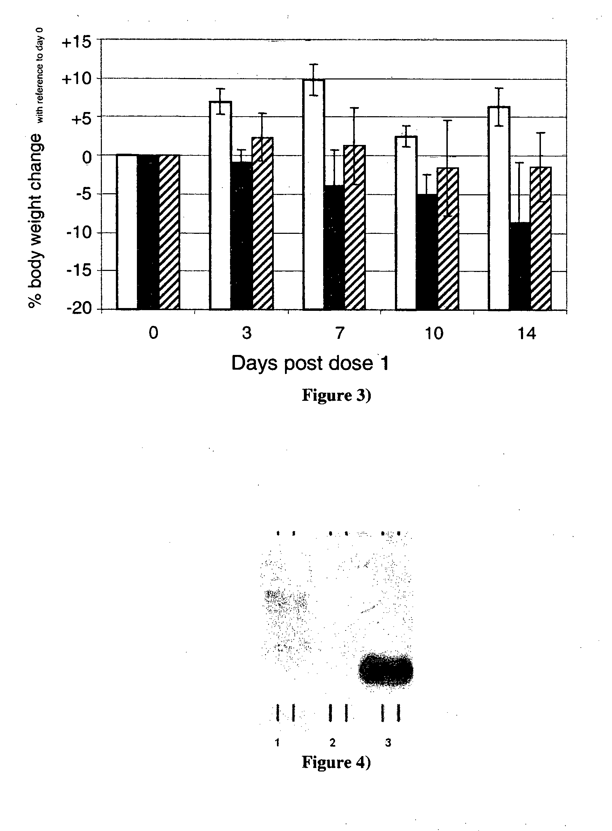

Figure 3) shows the calculated % body weight hanges (compared to day 0 (day of first dose) body weight) of individual mice as an average (bar) and standard deviation (error bars) with reference to dosing group (open bar ⬚ from mice dosed with a) rehydrated from freeze dried, with water for injection, liposomal siRNA duplex ID1, closed bar ▮ from mice dosed with b) rehydrated from freeze dried, with water for injection, siRNA duplex ID1 and hatched bar ▨ from mice dosed with c) rehydrated from freeze dried, with water for injection, siRNA duplex ID2)

Comments:-

The liposomal composition after rehydration were small in nature < 200 nm, such that in excess of 50% of the dose mass would be expected to pass through a 0.2 $\mu$m filter. Additionally over 50% (55.2 %) of the siRNA duplex (ID1) was associated with the liposomal particles, in a manner such that it was not recovered by ultracentrifugation in a wash buffer. The siRNA recovered from the ultracentrifugation wash buffer had an UV 260nm / UV 280 nm of 2.00 (=1.120 Abs / 0.558 Abs), indicative of a high siRNA purity.

Furthermore, the siRNA duplexes are both disclosed in Judge, *et al.*, *op.* cit. Furthermore, the siRNA duplex ID 1 is described in United States Patent Application 20060105976:-

| Examplary iRNA agents to target ApoB | | | | | |
|---|---|---|---|---|---|
| SEQ. ID No. | Sequence sense strand[a] | SEQ. ID No. | Sequence antisense strand[a] | Duplex descriptor[a] | Agent number |
| 97 | gucaucacacugaauaccaau | 98 | auugguauucagugugaugacac | AL-DUP 5048 | 11 |

In that specification the activity of the duplex AL-DUP 5048 (siRNA duplex ID 1, this example) is confirmed in an assay using NmuLi cells (normal murine liver, ATCC Number: CRL-1638), to reduce murine ApoB mRNA levels 72. +-.9%, and ApoB protein expression.

The siRNA duplex ID 2 is described in US-A-2007218122:-

ApoB Mismatch (mm) 5'-GUGAUCAGACUCAAUACGAAU-3' (SEQ ID NO:176) 3'-CACACUAGUCUGAGUUAUGCUUA-5' (SEQ ID NO:177) and is known to have no impact on mRNA production or translation. Judge, *et*

*al.*, show that this mismatched dsRNA when entrapped in cationic liposomes causes some weight loss in mice, though less than the encapsulated ID 1 siRNA.

Biochemically *(in vivo)* the ApoB protein forms a complex with triacylglycerols and cholesteryl esters encased in a sphere made up of phospholipid, plus non-esterified cholesterol. The LDL complex is the principal vehicle for delivering cholesterol to body tissues via the blood. An expected outcome of reduced ApoB protein expression would be a decrease in the blood concentration of LDL particles, since ApoB is an essential protein in the formation of LDL particles.

Indeed, Judge, et al., (2006) Molecular Therapy Vol. 13, No. 3, p494 - 505, commented that: "Functional silencing of apoB expression was reflected in significant reductions in serum cholesterol that also correlated with the relative potency of mRNA and protein knockdown" . In this exemplification we used the quantification of LDL particles as a correlate measure of specific siRNA efficacy. Duplex ID 1 as previously mentioned has the potential to reduce murine ApoB mRNA levels and ApoB protein expression, whilst duplex 2 was not expected to have any effect on ApoB, as shown by Judge, *et al.*

The results from the LDL / HDL cholesterol quantification system, (Figures 1a), 1b) and 2)) show the presence of three electrophoretically distinct cholesterol containing bands. These can be identified as: 1) the high-density lipoproteins (HDL) band, located closest to the origin, 2) the very low-density lipoprotein (VLDL) band, located second closest to the origin and 3) the low-density lipoprotein (LDL) band, located furthest from the origin (see reference gel shown in Figure 4). A few other minor bands are unidentified; however on the basis of the specificity of the kit employed, these can be assigned as cholesterol containing.

Figure 2), densitometry scans of the agarose gels from the serum sample analysis from mice dosed with :- c) rehydrated from freeze dried, with water for injection, siRNA duplex ID2, show no substantial changes in the LDL / HDL particle distribution profile at any time (days) after dosing. The profile stays consistently dominated by LDL particles, with only a minor part of serum cholesterol associated with HDL particles.

Figure 1b), densitometric scans of the agarose gels from the sera sample analysis from mice dosed with:- b) rehydrated from freeze dried, with water for injection, siRNA duplex ID1 without liposomal formulation, show no substantial changes in the LDL / HDL particle distribution profile at any time (days) after dosing. The profile stays consistently dominated by LDL particles, with only a minor part of serum cholesterol associated with HDL particles. As previously described the duplex ID 1 has the potential to reduce murine ApoB mRNA levels and ApoB protein expression, thus consequently LDL particles but this response is not seen in these experiments with naked siRNA. Comparison of Figures 2) and 1b) appears to show no substantial changes in the LDL / HDL particle distribution profile at any time (days) after dosing of either siRNA product (ID 1 and ID2).

Figure 1a), densitometric scans of the agarose gels from the serum sample analysis from mice dosed with :- a) rehydrated from freeze dried, with water for injection, liposomal siRNA duplex ID1, shows substantial changes in the LDL / HDL particle distribution profile at any time (days) after dosing. The LDL particle band is no longer visible at day 3, after 2 doses of liposomal siRNA duplex ID1, and starts to appear again by day 5. By day 7, equivalent to 4 days after the third dose liposomal siRNA duplex ID1, the LDL / HDL particle distribution profile is not substantially different from the day 0 profile (before dosing). Thus the response seen is transient and normal ApoB / LDL returns by day 7 after dosing by day 7.

Comparison of Figures 1 a) and 1b) highlights the substantial changes in the LDL / HDL particle distribution profile after dosing of the identical siRNA product (ID 1), with ( Figure 1a) ) and without (Figure 1b) ) liposomal composition. Indeed as noted in the absence of liposomal composition the siRNA duplex ID 1 appears ineffective, whilst in the liposomal composition described it is clearly effective.

Additionally, examination of the body weights of the mice following dosing Figure 3) indicates that; 1) both the non liposomal composition siRNA duplexes (ID 1 and 2) show a trend of decrease in body weight, 2) the liposomal composition siRNA duplex (ID 1) show a trend of increase in body weight (this is the expected normal pattern in untreated mice of this age group (4-6 weeks old)), and 3) the weight profile after dosing of the identical siRNA product (ID 1), with (Figure 2a)) and without (Figure 2b)) liposomal composition is statistically different (Mann-Whitney test, $P<0.05$) for all days post day 1.

Body weight is routinely used as an indicator of health, well-being and as a surrogate marker of toxicity, thus the non liposomal composition siRNA duplexes (ID 1 and 2) appear harmful, inducing weight loss. Whilst formulation of an siRNA duplex (ID 1) with a liposomal composition, eliminates this harmful effect.

In conclusion, the exemplification demonstrates an effective siRNA liposomal formulation, in mice, following intravenous dosing siRNA within liposomes with the added benefit of improved safety.

Example 2 - Preparation of Multi-Lamellar Vesicles

The preparation of multilamellar vesicles (MLV's), containing siRNA are exemplified as follows. Briefly, small unilamellar vesicles (SUV liposomes), 0.2 μm filtered, prepared with 100% DMPC (dimyristoyl phosphatidylcholine, Empirical Formula: C36H72NO8P; CAS# 18194-24-6) in solution were mixed with siRNA (duplex) material also in solution. Following the mixing of the preceding solutions, sucrose as a dry mass powder was added. After solubilisation of the sucrose mass the solution was frozen at -80°C and subsequently freeze dried, under high vacuum. The final MLV liposomal product containing siRNA, was formed by rehydration of the freeze dried material with water akin to the DRV (dried-rehydrated vesicle) method described in Example 1. The follow mass composition were prepared as described :- mass composition= F1 Liposome SUV's 5 %, siRNA 0.35% and sucrose 64.65%, F2 Liposome SUV's 26%, siRNA 0.26% and sucrose 73.74%.

The resultant rehydrated MLV liposomal formulations F1-F4 (inclusive) above, were assayed on a Malvern Zetasizer Nano (ZS) dynamic light scattering (DLS) instrument to yield data on particle size characteristics and zeta potential. The rehydrated formulations were diluted in water and assayed for particle size characteristics using square polystyrene sizing cuvette and assayed for zeta potential using a clear folded capillary zeta cell. All measurements were performed using automatic optimal criteria as determined by the instrument. The following particle size characteristics and zeta potential results for the respective compositions were based on duplicate rehydrated samples. For F1 the Zave particle size was 503 / 301 nm (duplicate), whilst the zeta potential was -2.55 / - 2:76mV (duplicate). For F2 the Zave particle size was 182 / 155nm (duplicate), whilst the zeta potential was 0.49 / -3.67mV (duplicate).

Additionally the resultant rehydrated MLV liposomal products rehydrated formulations F1 and F2 above, were assayed for siRNA material associated with the liposomes by differential centrifugation. The liposomes are sedimented by centrifugation and non-liposomal associated siRNA material remains in the supernatant. Validation experimentation with both individual components and mixtures, had previously indicated more than 95% liposome sedimentation and less than 5% siRNA sedimentation. Quantification of non-associated siRNA in the supernatant after centrifugation was carried out fluorometrically. The residue was assumed to be associated with liposomes. The following siRNA association values as a percentage of the total siRNA for the respective compositions were based on duplicate rehydrated material. The results for % siRNA liposomally associated were for F1 51 / 33% (duplicate) and for F3 19 / 16% (duplicate).

The higher level of sugar reduces the size of liposomes formed but also seems to reduce the entrapment yield.

**Claims**

1.  A method of preparing a liposomal siRNA composition comprising the steps of:

    i) providing an aqueous suspension of empty liposomes having an average diameter in the range 20 to 200 nm and formed of neutral liposome forming components;
    ii) mixing into the suspension double-stranded siRNA and sugar in an amount in the range 0.1 to 10.0% by weight based on the weight of liposome-forming components to form a mixed suspension, and in which the weight ratio of sugar to liposome forming components is in the range 0.75:1 to 7:1; and
    iii) dehydrating the mixture formed in step ii) to form a dehydrated composition,

    wherein liposomes formed by rehydrating the dehydrated composition have an average diameter in the range 100 to 500 nm and wherein the siRNA and sugar are each entrapped in the intravesicular space of the liposomes.

2.  A method according to claim 1, in which the sugar is a monosaccharide or a disaccharide.

3.  A method according to claim 2, in which the sugar is a disaccharide.

4.  A method according to claim 3, in which the sugar is sucrose.

5.  A method according to any preceding claim, in which the liposome-forming components comprise a phosphatidylcholine and optionally a phosphatidyl ethanolamine, and/or optionally cholesterol.

6.  A method according to any preceding claim, in which the weight ratio of sugar to liposome-forming components is in the range 0.9:1 to 5:1.

7.  A method according to any preceding claim, in which the empty liposomes of step i) have an average diameter in the range 25nm to 90nm.

8.  A method according to any preceding claim, in which step iii) is lyophilisation.

9.  A method according to any preceding claim, in which the weight ratio of sugar to liposome-forming components is in the range 1:1 to 6:1.

10. A method according to claim 9, in which the weight ratio of sugar to liposome-forming components is in the range 1:1 to 5:1.

11. A method according to any preceding claim, in which the empty liposomes of step i) have an average diameter in the range 50 to 90 nm.

12. A method according to any preceding claim, comprising the further step of:

    iv) rehydrating the dehydrated composition to form a rehydrated liposomal composition, wherein the siRNA and sugar are entrapped in the intravesicular space of the liposomes.

13. A method according to claim 12, in which the liposomes of the rehydrated composition have an average diameter in the range 100 to 200 nm.

14. A method according to any of claims 12 to 13, in which the rehydrated composition is subjected to microfluidisation.

15. A powder obtainable by the method of claims 1-7, or claims 9-11, wherein the dehydrating is carried out by spray drying.

16. An aqueous suspension of liposomes obtainable by the method of claims 1-11, comprising the further step of:

    iv) rehydrating the dehydrated composition to form a rehydrated liposomal composition, wherein the siRNA and sugar are each entrapped in the intravesicular space of the liposomes.

17. An aqueous suspension of liposomes, wherein the siRNA and sugar are each entrapped in the intravesicular space of the liposomes obtainable by the method of claims 12-14.


**Patentansprüche**

1.  Ein Verfahren zur Herstellung einer liposomalen siRNA Zusammensetzung umfassend die Schritte:

    i) Bereitstellen einer wässrigen Suspension von leeren Liposomen mit einem durchschnittlichen Durchmesser im Bereich von 20 bis 200 nm, die durch neutrale liposom-bildende Komponenten gebildet sind;
    ii) Beimischen doppelsträngiger siRNA und Zucker zur Suspension in einer Menge im Bereich von 0.1 bis 10.0 Gew.-% basierend auf dem Gewicht der liposom-formenden Komponenten, um eine gemischte Suspension zu bilden, und in welcher das Gewichtsverhältnis von Zucker zu liposom-formenden Komponenten im Bereich von 0.75:1 bis 7:1 ist; und
    iii) Dehydrieren der in Schritt ii) gebildeten Mischung, um eine dehydrierte Zusammensetzung zu bilden, wobei

    Liposomen, die durch Re-hydrieren der dehydrierten Zusammensetzung gebildet werden, einen durchschnittlichen Durchmesser im Bereich von 100 bis 500 nm aufweisen und wobei die siRNA und Zucker jeweils im intravesikulären Raum der Liposomen eingeschlossen sind.

2.  Ein Verfahren nach Anspruch 1, in welchem der Zucker ein Monosaccharid oder ein Disaccharid ist.

3.  Ein Verfahren nach Anspruch 2, in welchem der Zucker ein Disaccharid ist.

4.  Ein Verfahren nach Anspruch 3, in welchem der Zucker Saccharose ist.

**5.** Ein Verfahren nach einem der vorangehenden Ansprüchen, in welchem die liposom-bildende Komponenten Phosphatidylcholin und optional Phosphatidylethanolamin, und/oder optional Cholesterol umfasst.

**6.** Ein Verfahren nach einem der vorangehenden Ansprüchen, in welchem das Gewichtsverhältnis von Zucker zu liposom-formenden Komponenten im Bereich von 0.9:1 bis 5:1 ist.

**7.** Ein Verfahren nach einem der vorangehenden Ansprüchen, in welchem die leeren Liposomen aus Schritt i) einen durchschnittlichen Durchmesser im Bereich von 25 nm bis 90 nm aufweisen.

**8.** Ein Verfahren nach einem der vorangehenden Ansprüchen, in welchem Schritt iii) Lyophilisation ist.

**9.** Ein Verfahren nach einem der vorangehenden Ansprüchen, in welchem das Gewichtsverhältnis von Zucker zu liposom-formenden Komponenten im Bereich von 1:1 bis 6:1 ist.

**10.** Ein Verfahren nach einem der vorangehenden Ansprüchen, in welchem das Gewichtsverhältnis von Zucker zu liposom-formenden Komponenten im Bereich von 1:1 bis 5:1 ist.

**11.** Ein Verfahren nach einem der vorangehenden Ansprüchen, in welchem die leeren Liposomen aus Schritt i) einen durchschnittlichen Durchmesser im Bereich von 50 bis 90 nm aufweisen.

**12.** Ein Verfahren nach einem der vorangehenden Ansprüchen, umfassend den weiteren Schritt:

iv) Re-hydrieren der dehydrierten Zusammensetzung, um eine re-hydrierte liposomale Zusammensetzung zu bilden, wobei die siRNA und der Zucker im intravesikulären Raum der Liposomen eingeschlossen sind.

**13.** Ein Verfahren nach anspruch 12, in welchem die Liposomen der re-hydrierten Zusammensetzung einen durchschnittlichen Durchmesser im Bereich von 100 bis 200 nm aufweisen.

**14.** Ein Verfahren nach Anspruch 12 oder 13, in welchem die re-hydrierte Zusammensetzung einer Mikrofluidisierung unterworfen wird.

**15.** Ein Pulver erhältlich durch das Verfahren der Ansprüche 1 bis 7 oder der Ansprüche 9 bis 11, wobei das Dehydrieren durch Sprühtrocknen ausgeführt wird.

**16.** Eine wässrige Liposomen-Suspension erhältlich durch das Verfahren der Ansprüche 1 bis 11, umfassen den weiteren Schritt:

iv) Re-hydrieren der dehydrierten Zusammensetzung, um eine re-hydrierte liposomale Zusammensetzung zu bilden, wobei die siRNA und der Zucker im intravesikulären Raum der Liposomen eingeschlossen sind.

**17.** Ein wässrige Liposomen-Suspension, wobei die siRNA und Zucker jeweils im intravesikulären Raum der Liposomen, erhältlich durch das Verfahren der Ansprüche 12-14, eingeschlossen sind.

## Revendications

**1.** Procédé de préparation d'une composition liposomiale d'ARNsi comprenant les étapes suivantes :

i) la fourniture d'une suspension aqueuse de liposomes vides ayant un diamètre moyen situé dans la plage de 20 à 200 nm et formés de composants formant des liposomes neutres ;
ii) le mélange dans la suspension d'ARNsi double brin et de sucre dans une quantité située dans la plage de 0,1 à 10,0 % en poids en se basant sur le poids de composants formant des liposomes pour former une suspension mixte, et dans laquelle le rapport pondéral sucre sur composants formant des liposomes se situe dans la plage de 0,75:1 à 7:1 ; et
iii) la déshydratation du mélange formé dans l'étape ii) pour former une composition déshydratée,

dans lequel les liposomes formés par réhydratation de la composition déshydratée ont un diamètre moyen situé dans la plage de 100 à 500 nm et dans lequel l'ARNsi et le sucre sont chacun renfermés dans l'espace intravésiculaire des liposomes.

**2.** Procédé selon la revendication 1, dans lequel le sucre est un monosaccharide ou un disaccharide.

**3.** Procédé selon la revendication 2, dans lequel le sucre est un disaccharide.

**4.** Procédé selon la revendication 3, dans lequel le sucre est le saccharose.

**5.** Procédé selon l'une quelconque des revendications précédentes, dans lequel les composants formant des liposomes comprennent une phosphatidylcholine et éventuellement une phosphatidyléthanolamine, et/ou éventuellement du cholestérol.

**6.** Procédé selon l'une quelconque des revendications précédentes, dans lequel le rapport pondéral sucre sur composants formant des liposomes se situe dans la plage de 0,9:1 à 5:1.

**7.** Procédé selon l'une quelconque des revendications précédentes, dans lequel les liposomes vides de l'étape i) ont un diamètre moyen situé dans la plage de 25 nm à 90 nm.

**8.** Procédé selon l'une quelconque des revendications précédentes, dans lequel l'étape iii) est une lyophilisation.

**9.** Procédé selon l'une quelconque des revendications précédentes, dans lequel le rapport pondéral sucre sur composants formant des liposomes se situe dans la plage de 1:1 à 6:1.

**10.** Procédé selon la revendication 9, dans lequel le rapport pondéral sucre sur composants formant des liposomes se situe dans la plage de 1:1 à 5:1.

**11.** Procédé selon l'une quelconque des revendications précédentes, dans lequel les liposomes vides de l'étape i) ont un diamètre moyen situé dans la plage de 50 à 90 nm.

**12.** Procédé selon l'une quelconque des revendications précédentes, comprenant l'étape supplémentaire suivante :

iv) la réhydratation de la composition déshydratée pour former une composition liposomiale réhydratée, dans laquelle l'ARNsi et le sucre sont renfermés dans l'espace intravésiculaire des liposomes.

**13.** Procédé selon la revendication 12, dans lequel les liposomes de la composition réhydratée ont un diamètre moyen situé dans la plage de 100 à 200 nm.

**14.** Procédé selon l'une quelconque des revendications 12 ou 13, dans lequel la composition réhydratée est soumise à une microfluidisation.

**15.** Poudre pouvant être obtenue par le procédé selon les revendications 1 à 7, ou les revendications 9 à 11, dans laquelle la déshydratation est réalisée par séchage par pulvérisation.

**16.** Suspension aqueuse de liposomes pouvant être obtenue par le procédé selon les revendications 1 à 11, comprenant l'étape supplémentaire suivante :

iv) la réhydratation de la composition déshydratée pour former une composition liposomiale réhydratée, dans laquelle l'ARNsi et le sucre sont chacun renfermés dans l'espace intravésiculaire des liposomes.

**17.** Suspension aqueuse de liposomes, dans laquelle l'ARNsi et le sucre sont chacun renfermés dans l'espace intravésiculaire des liposomes pouvant être obtenus par le procédé selon les revendications 12 à 14.

**Figure 1a)**

**Figure 1b)**

**Figure 2)**

Figure 3)

Figure 4)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9810748 A
- WO 2004004758 A
- WO 9965465 A
- US 20070172430 A, Brito
- WO 2006113679 A
- US 20080063701 A, Keller

- US 20080089932 A, Panzner
- US 20060105976 A
- US 2007218122 A
- EP 2009064848 W
- EP 08168696 A

### Non-patent literature cited in the description

- **SPAGNOU et al.** *Biochemistry,* 2004, vol. 43, 13348-13356
- **JUDGE et al.** *Molecular Therapy,* 2006, vol. 13 (3), 494-505
- **KIRBY ; GREGORIADIS et al.** *Bio/technology,* 1984, 979-984
- **LANDEN et al.** *Cancer Research,* 2005, vol. 65 (15), 6910-6918
- **HALDER et al.** *Clin. Cancer Res.,* 2006, vol. 12 (16), 4916-4924

- **PONS et al.** *International Journal of Pharmaceutics,* 1993, vol. 95, 51-56
- **BATZRI ; KORN.** *Biochimica et Biophysica Acta,* 1973, vol. 298, 1015-1019
- **MAITANI et al.** *International Journal of Pharmaceutics,* 2008, vol. 356, 69-75
- **JUDGE et al.** *Molecular Therapy,* 2006, vol. 13 (3), 494-505